# EUROPEAN PATENT SPECIFICATION

(11) **EP 3 621 585 B1**
(45) Date of publication and mention of the grant of the patent: **27.01.2021**
(21) Application number: 18716282.1
(22) Date of filing: 10.04.2018
(51) Int. Cl.: A61K 8/66, A61K 8/73, A61Q 11/00

(54) **ORAL CARE COMPOSITIONS**
MUNDPFLEGEZUSAMMENSETZUNGEN
COMPOSITIONS DE SOINS ORAUX

(30) Priority: 11.05.2017 EP 17170644
(43) Date of publication of application: 18.03.2020
(73) Proprietor: Unilever PLC, London, Greater London EC4Y 0DY (GB); Unilever N.V., 3013 AL Rotterdam (NL)
(72) Inventor: BARFOOT, Richard, Jonathan, Bebington Wirral Merseyside CH63 3JW (GB); PERISSINOTO, Martina, 30174 Venice (IT)
(74) Representative: Tansley, Sally Elizabeth
(86) International application number: PCT/EP2018/059161
(87) International publication number: WO 2018/206211

(56) References cited:
- EP-A1- 3 192 497
- CN-A- 101 721 324
- JP-B2- 3 489 353
- US-A- 5 000 939
- OLSSON J ET AL: "Modulation of bacterial binding to salivary pellicle by treatment with hydrophilizing compounds", ARCHIVES OF ORAL BIOLOGY, PERGAMON PRESS, OXFORD, GB, vol. 35, 1 January 1990 (1990-01-01), pages S137-S140, XP027255160, ISSN: 0003-9969 [retrieved on 1990-01-01]
- VACCA SMITH W H BOWEN A M: "The Effects of Milk and kappa Casein on Salivary Pellicle Formed on Hydroxyapatite Discs in situ", CARIES RESE, S. KARGER AG, BASEL, CH, vol. 344, 1 January 2000 (2000-01-01), pages 88-93, XP008157287, ISSN: 0008-6568
- HANNIG M ET AL: "Protective effect of the in situ formed short-term salivary pellicle", ARCHIVES OF ORAL BIOL, PERGAMON PRESS, OXFORD, GB, vol. 49, no. 11, 1 November 2004 (2004-11-01), pages 903-910, XP004558415, ISSN: 0003-9969

## Description

### Field of the Invention

The present invention is concerned with oral care compositions. More particularly, the present invention is concerned with oral care compositions that enhance and/or maintain the dental pellicle.

The dental pellicle is a protein film that forms on the surface of teeth. It has many functions within the oral cavity, such as lubrication of oral surfaces, reduction of demineralisation and facilitation of remineralisation, barrier function, resistance to acid challenges and also modulation of bacterial adherence.

Pending EP patent application no. 16150969.0 discloses a composition comprising an enzyme and carrageenan for use in a method to maintain and/or enhance the mass of the salivary pellicle.

The dental pellicle can be removed when the teeth are brushed. The present application relates to compositions that clean the teeth and enhance the dental pellicle.

### Description of the Invention

The present invention relates to a composition comprising an enzyme and carrageenan in which the carrageenan comprises at least 50 wt% iota carrageenan and the enzyme comprises anamylase, an oxidase with oxygen as acceptor, a haloperoxidase or mixtures thereof.

The present invention further relates to a composition for use in enhancing the salivary pellicle, the composition comprising an enzyme and carrageenan in which the carrageenan comprises at least 50 wt% iota carrageenan and the enzyme comprises an amylase, an oxidase with oxygen as acceptor, a haloperoxidase or mixtures thereof.

### Detailed Description of the Invention

The present invention relates to a composition that deposits on the existing pellicle layer and thus helps protect the pellicle. It is thought that this deposition results in a change in the surface of the pellicle and helps build up an increased or enhanced pellicle layer. This increase in mass of the pellicle is, in the context of the present invention, seen as an enhancement.

Compositions according to the invention comprise carrageenan in which the carrageenan comprises at least 50 wt% of iota carrageenan.

In one preferred embodiment the composition the carrageenan is a mixture of components further comprising lambda carrageenan, kappa carrageenan or mixtures thereof.

Preferably the wt% of the lambda carrageenan within the carrageenan mix is below 50 wt%.

Preferably the wt% of kappa carrageenan within the carrageenan mix is below 50 wt%.

Preferably the weight ratio of iota carrageenan to lambda carrageenan is from 10:1 to 5:1.

Preferably the weight ratio of iota carrageenan to kappa carrageenan is from 10:1 to 5:1.

If 3 components are present in the carrageenan mix as described above it is preferred that iota carrageenan is present at a weight ratio greater than the sum of the other two components. Preferably the weight ratio of iota carrageenan to kappa carrageenan to lambda carrageenan is from 7:2:2 to 10:1 :1.

Preferably the level of carrageenan is from 0.05 wt% to 1.5 wt% of the total composition, more preferably from 0.1 wt% to 1.0 wt%.

The composition of the invention comprises an enzyme selected from the following groups:
i) an amylase, most preferably amylglucosidase;
ii) an oxoreductase acting on OH groups of donors, more preferably an oxidase with oxygen as acceptor, most preferably glucose oxidase;
iii) a hemeperoxidase, more preferably a haloperoxidase, most preferably a lactoperoxidase.

It is particularly preferred if the enzyme comprises amyloglucosidase.

A preferred embodiment comprises a mixture of 2 of the above groups, preferably 3 of the above groups, most preferably a mixture of amylglucosidase, and lactoperoxidase.

Preferably the total level of enzyme is from 0.01 wt% to 2wt% of the total composition. More preferably from 0.1 wt% to 1.5 wt% of the total composition.

Preferably the total level of amylase, particularly amylglucosidase, in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition.

Preferably the weight ratio of amylaseto other enzymes in the composition is greater than 1:1, more preferably greater than 3:2.

Preferably the weight ratio of total carrageenan (especially at preferred ratios) to enzymes is greater than 1:2, more preferably greater than 1:1, most preferably greater than 3:2.

Compositions of the invention may also comprise gums such as xanthan gum.

The total natural gums are present at levels from 0.1 wt% to 4 wt%, more preferably from 0.1 to 3 wt% of the total composition.

Additionally, other structurant agents such as binders and thickening agents may be present. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose).

Compositions of the invention may also comprise other proteins such as lysozymes, lactoferrins and colostrum. Preferably these other proteins are present at total levels from 0.001 to 0.5 wt% of the total composition, preferably from 0.005 to 0.1 wt% of the total composition.

Furthermore, the oral care composition comprises a nonionic surfactant in an amount of from 0.2 to 5% by weight based on the total weight of the dentifrice. Preferred nonionic surfactants are polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.5 to 7 wt% of the total composition, more preferably from 1 to 5 wt% of the total composition.

Although other surfactants may be present it is preferred if they are limited to levels below 0.5 wt% of the total composition preferably less than 0.1 wt% of the total composition. It is particularly preferred if the composition is substantially free of anionic surfactants such that it comprises less than 0.05 wt% of anionic surfactant.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically, the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60 by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonates, colloidal magnesium aluminium silicates and mixtures thereof.

Compositions of the present invention may also contain further optional ingredients customary in the art such as fluoride ion sources, anticalculus agents, buffers, flavouring agents, bleaching agents, sweetening agents, colouring agents, opacifying agents, preservatives, antisensitivity agents and antimicrobial agents.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples:

### Examples

The following Examples were prepared

**Table 1a**

| **Ingredient** | **Example N.** | | | | |
|---|---|---|---|---|---|
| | **1** | **2** | **3** | **4** | **5** |
| *Iota Carrageenan* | 0.0000% | 0.0000% | 0.0979% | 0.0000% | 0.0000% |
| *Lambda Carrageenan* | 0.0000% | 0.0979% | 0.0210% | 0.0000% | 0.0490% |
| *Kappa Carrageenan* | 0.0000% | 0.0419% | 0.0210% | 0.0000% | 0.0210% |
| *Lactoperoxidase* | 0.0000% | 0.0000% | 0.0000% | 0.0020% | 0.0020% |
| *Glucose Oxidase* | 0.0272% | 0.0272% | 0.0272% | 0.0000% | 0.0000% |
| *Amyloglucosidase* | 0.0000% | 0.0000% | 0.0000% | 0.0000% | 0.0000% |
| *Water* | 99.9728% | 99.8330% | 99.8329% | 99.9980% | 99.9280% |

| | | | | | |
|---|---|---|---|---|---|
| *Table 1a & 1b. Concentration (% w*/*w) of each ingredient for all examples tested by QCM-D.* | | | | | |

**Table 1b**

| **Ingredient** | **Example N.** | | | |
|---|---|---|---|---|
| | **6** | **7** | **8** | **9** |
| *Iota Carrageenan* | 0.0490% | 0.0000% | 0.0000% | 0.0941% |
| *Lambda Carrageenan* | 0.0105% | 0.0000% | 0.0941% | 0.0202% |
| *Kappa Carrageenan* | 0.0105% | 0.0000% | 0.0403% | 0.0202% |
| *Lactoperoxidase* | 0.0020% | 0.0000% | 0.0000% | 0.0000% |
| *Glucose Oxidase* | 0.0000% | 0.0000% | 0.0000% | 0.0000% |
| *Amyloglucosidase* | 0.0000% | 4.0000% | 0.8000% | 0.8000% |
| *Water* | 99.9280% | 96.0000% | 99.0656% | 99.0655% |

| | | | | |
|---|---|---|---|---|
| *Table 2a & 1b. Concentration (% w*/*w) of each ingredient for all examples tested by QCM-D.* | | | | |

### QCM-D of Salivary Pellicle

Quartz Crystal Microbalance with Dissipation monitoring (QCM-D) is a technique that allows nanoscale mass and structural changes to be measured. The measurements are based on changes in vibration frequency of a quartz crystal sensor in response to interaction (adsorption) and reactions at the sensor surface. QCM-D has been previously applied to understand salivary pellicle on a range of substrates.

### Materials

Stimulated human saliva was collected from different healthy donors and diluted 1:5 in deionised water.

Carrageenans were weighed to the required ratios and dispersed in deionised water. The enzymes were homogeneously mixed either with water, or with a kappa and lambda carrageenan mix, or with an iota, kappa and lambda carrageenan combination.

### Method

Quartz crystal microbalance with dissipation monitoring (QCM-D) measurements were performed on gold-coated quartz crystals with a fundamental resonant frequency of 5MHz. Treatments were either lactoperoxidase, glucose oxidase or amyloglucosidase in water, in a kappa and lambda carrageenan mix, and in a combination of iota, kappa and lambda carrageenan.

Gold-coated QCM-D crystals were subjected to the following regime: deionised water, diluted saliva, deionised water, enzyme mix, deionised water. diluted saliva and deionised water. The experiments were conducted at a temperature of 21°C and repeated at least twice.

The Sauerbrey equation was used to approximate the mass of the salivary pellicle adsorbed for each sample as measure of the areal mass (ng/cm²). Salivary pellicle growth (ng/cm²) was calculated as a difference between the areal mass at the water rinsing step after the second saliva addition and the areal mass at the water washing step following the first introduction of saliva. Mean and standard deviation were determined for each enzyme combination. Statistical analysis (JMP v.11.0, SAS) was carried out using analysis of variance (ANOVA) and Tukey mean comparison test and the level of statistical significance was set at p<0.05.

**Table 2**

| **Enzyme** | **Example N.** | **Pellicle Growth (ng/cm²)** | | **p-value** |
|---|---|---|---|---|
| | | **Mean** | **St. Dev.** | |
| *Lactoperoxidase* | **1** | 90.67 | 30.35 | 0.0011 |
| | **2** | 103.33 | 33.20 | |
| | **3** | 388.33 | 87.76 | |
| *Glucose Oxidase* | **4** | -8.67 | 100.17 | 0.0096 |
| | **5** | 590.67 | 289.47 | |
| | **6** | 728.67 | 175.10 | |
| *Amyloglucosidase* | **7** | 30.50 | 7.78 | <0.0001 |
| | **8** | 839.00 | 156.98 | |
| | **9** | 1178.00 | 79.54 | |

QCM-D measurements show that by changing the enzymes and the ratio of the types of carrageenan present in the toothpaste the effect on pellicle growth can be modulated leading to a higher increase in the pellicle mass.

## Claims

1. A composition comprising an enzyme and carrageenan in which the carrageenan comprises at least 50 wt% iota carrageenan and the enzyme comprises an amylase, an oxidase with oxygen as acceptor, a haloperoxidase or mixtures thereof.

2. A composition according to claim 1 in which the carrageenan further comprises lambda carrageenan, kappa carrageenan or mixtures thereof.

3. An oral care composition according to any preceding claim in which the wt% of the lambda carrageenan within the carrageenan mix is below 50 wt%.

4. An oral care composition according to any preceding claim in which the wt% of kappa carrageenan within the carrageenan mix is below 50 wt%.

5. An oral care composition according to any preceding claim in which the amylase is amyloglucosidase.

6. A composition according to any preceding claim in which the haloperoxidase comprises lactoperoxidase.

7. A composition according to any preceding claim, which comprises a nonionic surfactant.

8. A composition according to any preceding claim in which the non-ionic surfactant is a polyethylene glycol ether of a fatty alcohol.

9. A composition according to any preceding claim, which comprises less than 0.05 wt% of anionic surfactant.

10. A composition according to any preceding claim, which is in the form of a dentifrice.

11. A composition for use in enhancing the salivary pellicle, the composition comprising an enzyme and carrageenan in which the carrageenan comprises at least 50 wt% iota carrageenan and the enzyme comprises an amylase, an oxidase with oxygen as acceptor, a haloperoxidase or mixtures thereof.

## Patentansprüche

1. Zusammensetzung, umfassend ein Enzym und Carrageenan, wobei das Carrageenan mindestens 50 Gew.-% Iota-Carrageenan umfasst und das Enzym eine Amylase, eine Oxidase mit Sauerstoff als Akzeptor, eine Haloperoxidase oder Mischungen davon umfasst.

2. Zusammensetzung nach Anspruch 1, wobei das Carrageenan ferner Lambda-Carrageenan, Kappa-Carrageenan oder Mischungen davon umfasst.

3. Mundpflegezusammensetzung nach irgendeinem vorhergehenden Anspruch, wobei die Gew.-% des Lambda-Carrageenans in der Carrageenan-Mischung unter 50 Gew.-% liegen.

4. Mundpflegezusammensetzung nach irgendeinem vorhergehenden Anspruch, wobei die Gew.-% von Kappa-Carrageenan in der Carrageenan-Mischung unter 50 Gew.-% liegen.

5. Mundpflegezusammensetzung nach irgendeinem vorhergehenden Anspruch, wobei die Amylase Amyloglucosidase ist.

6. Zusammensetzung nach irgendeinem vorhergehenden Anspruch, wobei die Haloperoxidase Lactoperoxidase umfasst.

7. Zusammensetzung nach irgendeinem vorhergehenden Anspruch, die ein nichtionisches Tensid umfasst.

8. Zusammensetzung nach irgendeinem vorhergehenden Anspruch, wobei das nichtionische Tensid ein Polyethylenglycolether von einem Fettalkohol ist.

9. Zusammensetzung nach irgendeinem vorhergehenden Anspruch, die weniger als 0,05 Gew.-% anionisches Tensid umfasst.

10. Zusammensetzung nach irgendeinem vorhergehenden Anspruch, die in Form eines Zahnreinigungsmittels vorliegt.

11. Zusammensetzung zur Verwendung zur Verbesserung der Speichelpellikel, wobei die Zusammensetzung ein Enzym und Carrageenan umfasst, wobei das Carrageenan mindestens 50 Gew.-% Iota-Carrageenan umfasst und das Enzym eine Amylase, eine Oxidase mit Sauerstoff als Akzeptor, eine Haloperoxidase oder Mischungen davon umfasst.

## Revendications

1. Composition comprenant une enzyme et du carraghénane, dans laquelle le carraghénane comprend au moins 50 % en masse de carraghénane iota et l'enzyme comprend une amylase, une oxydase avec de l'oxygène comme accepteur, une haloperoxydase ou des mélanges de celles-ci.

2. Composition selon la revendication 1, dans laquelle le carraghénane comprend de plus du carraghénane lambda, carraghénane kappa ou des mélanges de ceux-ci.

3. Composition pour le soin oral selon l'une quelconque des revendications précédentes, dans laquelle le % en masse du carraghénane lambda dans le mélange de carraghénane est inférieur à 50 % en masse.

4. Composition pour le soin oral selon l'une quelconque des revendications précédentes, dans laquelle le % en masse de carraghénane kappa dans le mélange de carraghénane est inférieur à 50 % en masse.

5. Composition pour le soin oral selon l'une quelconque des revendications précédentes, dans laquelle l'amylase est l'amyloglucosidase.

6. Composition pour le soin oral selon l'une quelconque des revendications précédentes, dans laquelle l'haloperoxydase comprend de la lactoperoxydase.

7. Composition selon l'une quelconque des revendications précédentes, qui comprend un tensioactif non ionique.

8. Composition selon l'une quelconque des revendications précédentes, dans laquelle le tensioactif non-ionique est un polyéthylène glycoléther d'un alcool gras.

9. Composition selon l'une quelconque des revendications précédentes, qui comprend moins de 0,05 % en masse de tensioactif anionique.

10. Composition selon l'une quelconque des revendications précédentes, qui est dans la forme d'un dentifrice.

11. Composition pour une utilisation dans la promotion de la pellicule salivaire, la composition comprenant une enzyme et du carraghénane, dans laquelle le carraghénane comprend au moins 50 % en masse de carraghénane iota et l'enzyme comprend une amylase, une oxydase avec de l'oxygène comme accepteur, une haloperoxydase ou des mélanges de celles-ci.
